(19) Europäisches Patentamt
European Patent Office
Office européen des brevets

(11) Publication number: **0 076 758**
**B1**

(12) **EUROPEAN PATENT SPECIFICATION**

(45) Date of publication of patent specification: **07.01.87**

(51) Int. Cl.[4]: **C 07 D 417/12,** C 07 D 403/12 // C07D205/08, A61K31/495, A61K31/425

(21) Application number: **82401804.8**

(22) Date of filing: **04.10.82**

# (54) 4-Ether derivatives of 2-azetidinone-1-sulfonic acids.

(30) Priority: **05.10.81 US 308229**

(43) Date of publication of application:
**13.04.83 Bulletin 83/15**

(45) Publication of the grant of the patent:
**07.01.87 Bulletin 87/02**

(84) Designated Contracting States:
**AT BE CH DE FR GB IT LI LU NL SE**

(56) References cited:
**GB-A-2 091 723**

(73) Proprietor: **E.R. Squibb & Sons, Inc.**
**Lawrenceville-Princeton Road**
**Princeton, N.J. 08540 (US)**

(72) Inventor: **Kronenthal, David R.**
**21 Oak Ridge Drive**
**Langhorne Pennsylvania (US)**
Inventor: **Breuer, Hermann**
**Sauerzapfstrasse 5**
**D-8411 Schoenhofen (DE)**

(74) Representative: **Maiffret, Bernard et al**
**Law Offices of William J. Rezac**
**49 avenue Franklin D. Roosevelt**
**F-75008 Paris (FR)**

**EP 0 076 758 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Antibacterial activity is exhibited by β-lactams having the formula

I

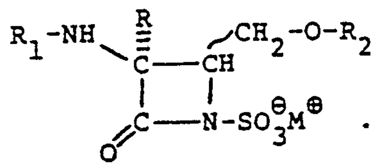

In formula I and throughout the specification the symbols are as defined below.

R is hydrogen or methoxy;

$R_1$ is acyl;

$R_2$ is alkyl, aralkyl, carboxyalkyl, alkoxycarbonylalkyl, $NH_2—(CH_2)_n—$, $HO—(CH_2)_n—$ or aryl wherein n is 2, 3 or 4; and

M is hydrogen or $M^{\oplus}$ is a cation, with the proviso that if M is hydrogen the $R_1$ or $R_2$ group contains a basic function.

Listed below are definitions of various terms used to describe the β-lactams of this invention. These definitions apply to the terms as they are used throughout the specification (unless they are otherwise limited in specific instances) either individually or as part of a larger group.

The term "aryl" refers to phenyl and phenyl substituted with 1, 2 or 3 amino ($—NH_2$), halogen, hydroxyl, trifluoromethyl, alkyl (of 1 to 4 carbon atoms), alkoxy (of 1 to 4 carbon atoms) or carboxyl groups.

The term "acyl" includes acyl groups which have been used in the past to acylate β-lactam antibiotics including 6-aminopenicillanic acid and derivatives and 7-aminocephalosporanic acid and derivatives; see, for example, *Cephalosporins and Penicillins,* edited by Flynn, Academic Press (1972), German Offenlegungsschrift 2,716,677, published October 10, 1978, Belgian patent 867,994, published December 11, 1978, United States patent 4,152,432, issued May 1, 1979, United States patent 3,971,778, issued July 27, 1976, United States patent 4,172,199, issued October 23, 1979, and British patent 1,348,894, published March 27, 1974. The portions of these references describing various acyl groups are incorporated herein by reference. The following list of acyl groups is presented to further exemplify the term "acyl"; it should not be regarded as limiting that term. Exemplary acyl groups are:

(a) Aliphatic groups having the formula

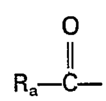

wherein $R_a$ is alkyl; cycloalkyl; alkoxy; alkenyl; cycloalkenyl; cyclohexadienyl; or alkyl or alkenyl substituted with one or more halogen, cyano, nitro, amino, mercapto, alkylthio, or cyanomethylthio groups.

(b) Carbocyclic aromatic groups having the formula

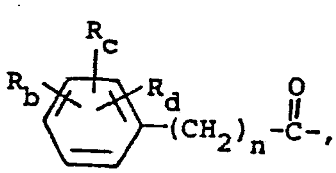,
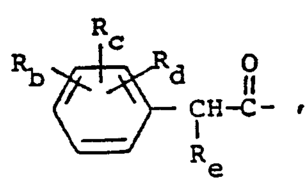,
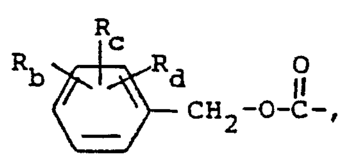,

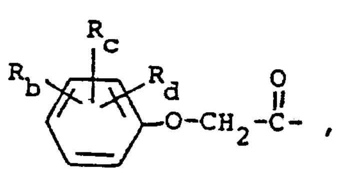,
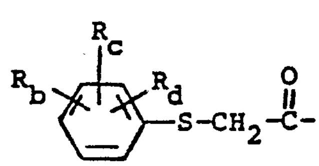 or
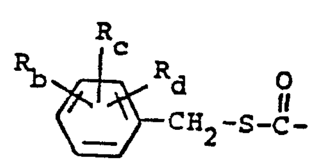

wherein n is 0, 1, 2 or 3; $R_b$, $R_c$, and $R_d$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_e$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio.

Preferred carbocyclic aromatic acyl groups include those having the formula

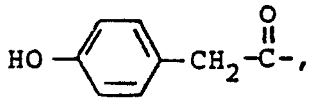 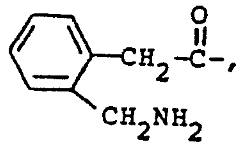 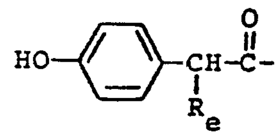

($R_e$ is preferably a carboxyl salt or sulfo salt) and

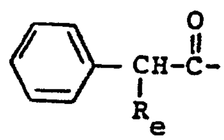

($R_e$ is preferably a carboxyl salt or sulfo salt).

(c) Heteroaromatic groups having the formula

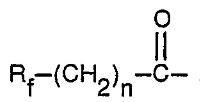 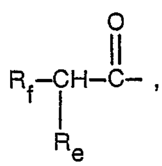 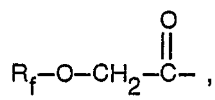 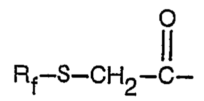 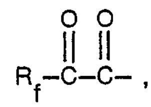

wherein n is 0, 1, 2 or 3; $R_e$ is as defined above; and $R_f$ is a substituted or unsubstituted 5-, 6- or 7-membered heterocyclic ring containing 1, 2, 3 or 4 (preferably 1 or 2) nitrogen, oxygen and sulfur atoms. Exemplary heterocyclic rings are thienyl, furyl, pyrrolyl, pyridinyl, pyrazolyl, pyrazinyl, thioazolyl, pyrimidinyl and tetrazolyl. Exemplary substituents are halogen, hydroxyl, nitro, amino, cyano, trifluoromethyl, alkyl of 1 to 4 carbon atoms, alkoxy of 1 to 4 carbon atoms, or

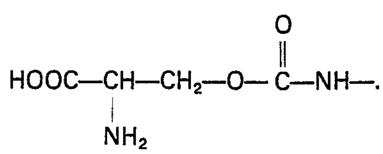

Preferred heteroaromatic acyl groups include those groups of the above formulas wherein $R_f$ is 2-amino-4-thiazolyl, 2-amino-5-halo-4-thiazolyl, 4-aminopyrimidin-2-yl, 5-amino-1,2,4-thiadiazol-3-yl, 2-thienyl, 2-furanyl, or 6-aminopyridin-2-yl.

(d) [[(4-Substituted-2,3-dioxo-1-piperazinyl)carbonyl]amino]arylacetyl groups having the formula

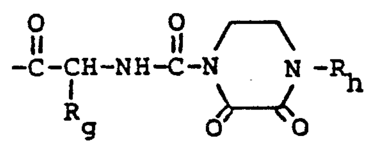

wherein $R_g$ is an aromatic group (including carbocyclic aromatics such as those of the formula

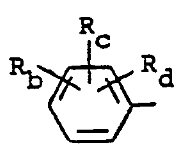

and heteroaromatics as included within the definition of $R_f$); and $R_h$ is alkyl, substituted alkyl (wherein the alkyl group is substituted with one or more halogen, cyano, nitro, amino or mercapto groups), arylmethyleneamino (*i.e.,* —N=CH—$R_g$ wherein $R_g$ is as defined above), arylcarbonylamino (*i.e.,*

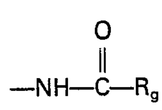

wherein $R_g$ is as defined above) or alkylcarbonylamino.

Preferred [[(4-substituted-2,3-dioxo-1-piperazinyl)carbonyl]amino]arylacetyl groups include those wherein $R_h$ is ethyl, phenylmethyleneamino or 2-furylmethyleneamino.

(e) (Substituted oxyimino)arylacetyl groups having the formula

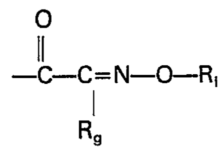

wherein $R_g$ is as defined above and $R_i$ is hydrogen, alkyl, cycloalkyl, alkylaminocarbonyl, arylaminocarbonyl (*i.e.,*

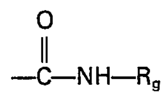

wherein $R_g$ is as defined above) or substituted alkyl (wherein the alkyl group is substituted with 1 or more halogen, cyano, nitro, amino, mercapto, alkylthio, aromatic group (as defined by $R_g$), carboxyl (including salts thereof), amido, alkoxycarbonyl, phenylmethoxycarbonyl, diphenylmethoxycarbonyl, hydroxyalkoxyphosphinyl, dihydroxyphosphinyl, hydroxy(phenylmethoxy)phosphinyl, or dialkoxyphosphinyl substitutents).

Preferred (substituted oxyimino)arylacetyl groups include those wherein $R_g$ is 2-amino-4-thiazolyl. Also preferred are those groups wherein $R_i$ is methyl, ethyl, carboxymethyl, 1-carboxy-1-methylethyl or 2,2,2-trifluoroethyl.

(f) (Acylamino)arylacetyl groups having the formula

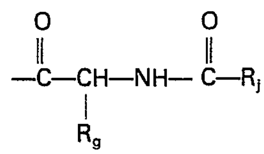

wherein $R_g$ is as defined above and $R_j$ is

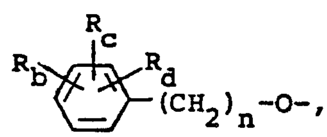

amino, alkylamino, (cyanoalkyl)amino, amido, alkylamido, (cyanoalkyl)amido,

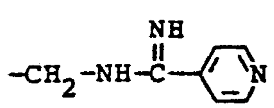

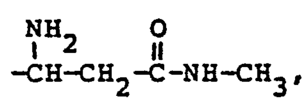

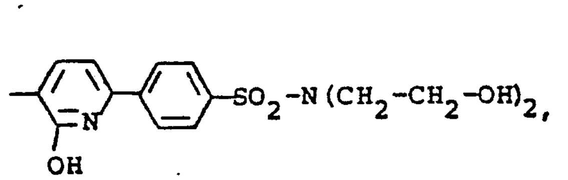

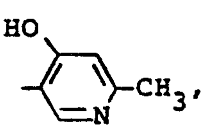

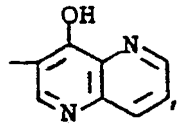

or

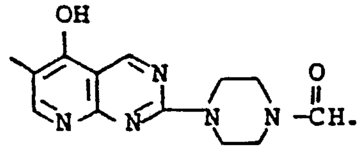

Preferred (acylamino)arylacetyl groups of the above formula include those groups wherein $R_j$ is amino or amido. Also preferred are those groups wherein $R_g$ is phenyl or 2-thienyl.

(g) [[[3-Substituted-2-oxo-1-imidazolidinyl]carbonyl]amino]arylacetyl groups having the formula

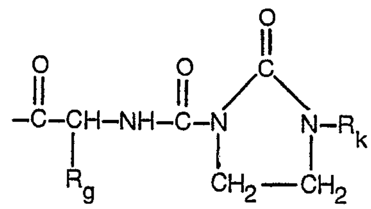

wherein $R_g$ is as defined above and $R_k$ is hydrogen, alkylsulfonyl, arylmethyleneamino (*i.e.*, $—N{=}CH—R_g$ wherein $R_g$ is as defined above),

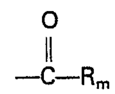

(wherein $R_m$ is hydrogen, alkyl or halogen substituted alkyl), aromatic group (as defined by $R_g$ above), alkyl or substituted alkyl (wherein the alkyl group is substituted with one or more halogen, cyano, nitro, amino or mercapto groups).

Preferred [[3-substituted-2-oxo-1-imidazolidinyl]carbonyl]amino]arylacetyl groups of the above formula include those wherein $R_g$ is phenyl or 2-thienyl. Also preferred are those groups wherein $R_k$ is hydrogen, methylsulfonyl, phenylmethyleneamino or 2-furylmethyleneamino.

The terms "alkyl" and "alkoxy" refer to both straight and branched chain groups having 1 to 10 carbon atoms preferably having 1 to 4 carbon atoms.

The terms "alkanoyl" and "alkenyl" refer to both straight and branched chain groups. Those groups having 2 to 10 carbon atoms are preferred.

The terms "cycloalkyl" and "cycloalkenyl" refer to groups having 3, 4, 5, 6 or 7 carbon atoms.

The term "cation", as used throughout the specification, refers to any positively charged atom or group of atoms. The "$—SO_3^{\ominus}M^{\oplus}$" substituent on the nitrogen atom of the β-lactams of this invention encompasses all sulfonic acid salts. Pharmaceutically acceptable salts are, of course, preferred, although other salts are also useful in purifying the products of this invention or as intermediates for the preparation of pharmaceutically acceptable salts. The cationic portion of the sulfonic acid salts of this invention can be obtained from either organic or inorganic bases. Such cationic portion includes, but is not limited to, the following ions: ammonium; substituted ammonium, such as alkylammonium (*e.g.*, tetra-n-butylammonium, referred to hereinafter as tetrabutylammonium); alkali metal, such as lithium, sodium and potassium; alkaline earth metal, such as calcium and magnesium; pyridinium, dicyclohexylammonium; hydrabaminium; benzathinium; N-methyl-D-glucaminium.

As set forth in formula I, and in the definitions following formula I, M can be hydrogen. Such compounds are often referred to in the art as "inner salts" by virtue of a positive and negative charge in the molecule.

This invention is directed to those β-lactams which have been described above, wherein the stereochemistry at the chiral center in the 3-position of the β-lactam nucleus is the same as the configuration at the carbon atom in the 6-position of naturally occurring penicillins (*e.g.*, penicillin G) and as the configuration at the carbon atom in the 7-position of naturally occurring cephamycins (*e.g.*, cephamycin C). However, these compounds can be prepared as enantiomeric mixtures containing both the active and inactive epimers. This invention encompasses the enantiomeric mixture, as well as any other composition containing the isomers with the absolute configuration specified above.

The β-lactams of formula I have activity against a range of gram-negative and gram-positive organisms. The compounds of this invention can be used as agents to combat bacterial infections (including urinary tract infections and respiratory infections) in mammalian species, such as domesticated animals (*e.g.*, dogs, cats, cows, horses, and the like) and humans.

For combating bacterial infections in mammals a compound of this invention can be administered to a mammal in need thereof in an amount of about 1.4 mg/kg/day to about 350 mg/kg/day, preferably about 14 mg/kg/day to about 100 mg/kg/day. All modes of administration which have been used in the past to deliver penicillins and cephalosporins to the site of the infection are also contemplated for use with the novel β-lactams of this invention. Such methods of administration include oral, intravenous, intramuscular, and as a suppository.

The compounds of formula I wherein $R_2$ is aryl can be prepared from an azetidine having the formula

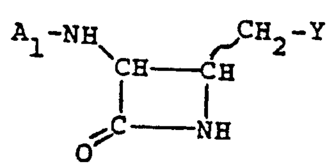

II

wherein Y is a leaving group such as iodine or *p*-toluenesulfonyloxy and $A_1$ is a nitrogen protecting group, *e.g.,* benzyloxycarbonyl, triphenylmethyl or *t*-butoxycarbonyl. Reaction of a compound of formula II with the appropriate phenolic compound having the formula

$$R_2'—OH \qquad III$$

wherein $R_2'$ is aryl, in the presence of base, *e.g.,* sodium hydride, yields the corresponding compound having the formula

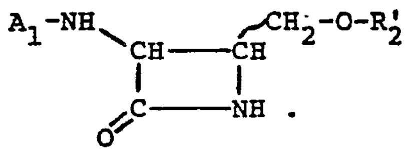

IV

Introduction of the sulfo substituent onto a compound of formula IV yields a compound having the formula

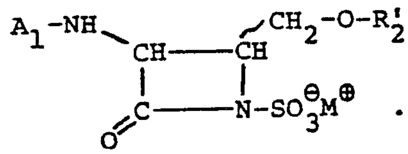

V

This is accomplished by reacting a compound of formula IV with a complex of pyridine and sulfur trioxide. The reaction can be run in an organic solvent or in a mixture of organic solvents, preferably a mixture of a polar solvent such as dimethylformamide and a halogenated hydrocarbon such as dichloromethane. This reaction yields a compound of formula V wherein $M^{\oplus}$ is pyridinium ion. Instead of using a pre-formed complex of pyridine and sulfur trioxide, the complex can be formed *in situ, e.g.,* using chlorosulfonyltrimethylsilyl ester and pyridine as reagents. Alternatively, a complex of dimethylformamide-sulfur trioxide, 2-picolinesulfur trioxide or 2,6-lutidine sulfur trioxide can be used.

Using conventional techniques (*e.g.,* ion-exchange resins, or ion-pair extraction) the pyridinium salt formed by the above procedure can be converted to other salts. These techniques can also be used to convert the products of formula I, or any of the intermediates described herein, to other salts.

A second method for introducing the sulfo group to the 1-position of an azetidine of formula IV comprises first silylating the compound and then subjecting the silated compound to a silyl interchange reaction. Exemplary silylating agents are monosilyltrifluoroacetamide, trimethylsilylchloride/triethylamine, and bis-trimethylsilyltrifluoroacetamide, and an exemplary reagent useful for the silyl interchange reaction is trimethylsilyl chlorosulfonate.

Deprotection of an azetidine of formula V yields a zwitterion having the formula

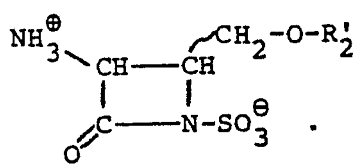

VI

The deprotection techniques used are conventional, and will depend on the particular protecting group ($A_1$) present. Treatment with acid (*e.g.,* formic acid or trifluoroacetic acid) cleaves a triphenylmethyl or a *t*-butoxycarbonyl protecting group. A benzyloxycarbonylamino protecting group can be cleaved by catalytic hydrogenation. Treatment with phosgene or phosphorous pentachloride cleaves an amide protecting group.

Conventional acylation techniques can be used to prepare the products of formula I (wherein R is hydrogen) from a zwitterion of formula VI. Exemplary acylation techniques include reaction with a carboxylic acid ($R_1$—OH), or corresponding carboxylic acid halide or carboxylic acid anhydride. The reaction with a carboxylic acid proceeds most readily in the presence of a carbodiimide and a substance capable of forming an active ester *in situ* such as N-hydroxybenzotriazole. In those instances wherein the acyl group ($R_1$) contains reactive functionality (such as amino or carboxyl groups) it may be necessary to first protect those functional groups, then carry out the acylation reaction, and finally deprotect the resulting product.

Those compounds of formula I wherein $R_2$ is alkyl, arylalkl, carboxyalkyl, alkoxycarbonylalkyl, aminoalkyl and hydroxyalkyl can be prepared from an azetidine having the formula

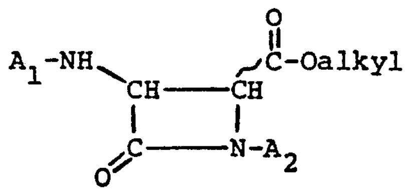

VII

wherein $A_2$ is a protecting group such as 4-methoxyphenyl, (2,4-dimethoxyphenyl)methyl or di(4-methoxyphenyl)methyl. Chemical reduction of a compound of formula VII using for example, sodium borohydride, yields the corresponding azetidine having the formula

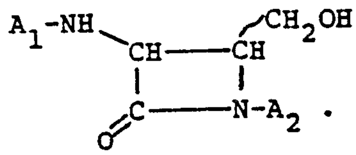

VIII

Alkylation of a compound of formula VIII yields the corresponding compound having the formula

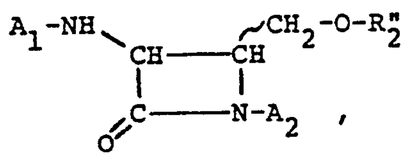

IX

wherein $R_2''$ is alkyl, arylalkyl or alkoxycarbonylalkyl. Alkylation can be accomplished by reacting a compound of formula VIII with the appropriate diazoalkane or substituted diazoalkane in the presence of a catalyst such as boron trifluoride etherate or rhodium (II) acetate.

Oxidative removal of the 1-substituent of a compound of formula IX yields the corresponding compound having the formula

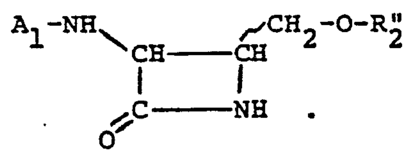

X

Ceric ammonium nitrate is the preferred oxidizing agent. When $A_2$ (compound of formula IX) is (2,4-dimethoxyphenyl)methyl or di(4-methoxyphenyl)methyl, trifluoroacetic acid-anisole can also be used. When $A_2$ is (2,4-dimethoxyphenyl)methyl, potassium persulfate can also be used.

Conversion of a compound of formula VIII to a product of formula I can be accomplished using the methodology described above for the conversion of a compound of formula IV to a product of formula I, yielding first a compound having the formula

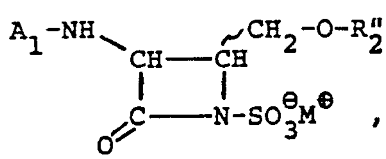

XI

then a zwitterion having the formula

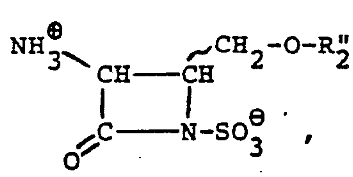

XII

and finally the desired product of formula I.

To prepare those compounds of formula I wherein $R_2$ is carboxyalkyl, a compound of formula X wherein $R_2''$ is alkoxycarbonyl is saponified to yield the corresponding compound having the formula

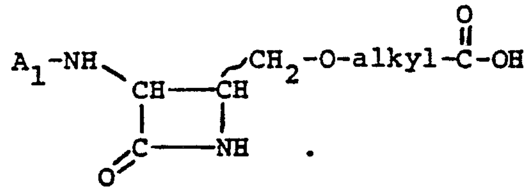

XIII

Prior to the introduction of the sulfo substituent in the 1-position of a compound of formula XIII it is necessary to first protect the carboxyl group. This can be accomplished, for example, by reacting a compound of formula XIII with diphenyl diazomethane to yield the corresponding compound having the formula

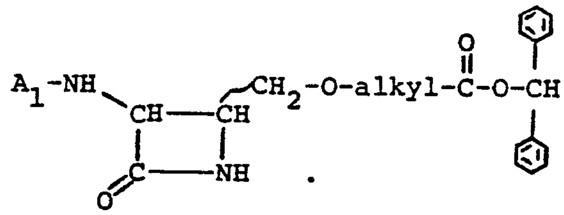

XIV

Introduction of a sulfo substituent to the 1-position of a compound of formula XIV can be accomplished using the procedures described above for the introduction of a sulfo substituent onto a compound of formula IV. The resulting compound has the formula

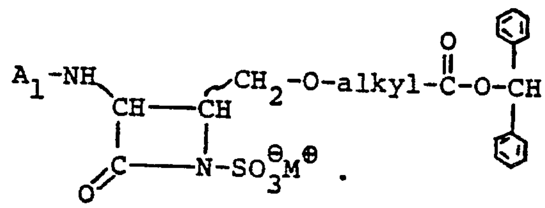

XV

Deprotection of a compound of formula XV using art-recognised techniques yields the corresponding zwitterion having the formula

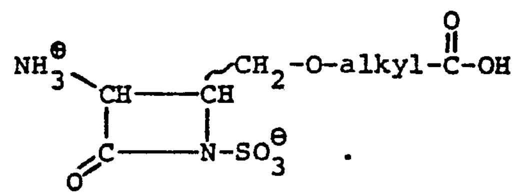

XVI

Acylation of a zwitterion of formula XVI to yield the corresponding product of formula I can be accomplished using the conventional acylation techniques described above.

To prepare those compounds of formula I wherein $R_2$ is $HO—(CH_2)_n—$, a compound of formula XI wherein $R_2''$ is alkoxycarbonylalkyl is selectively reduced (using, for example, a chemical reducing agent such as sodium borohydride) to yield a compound having the formula

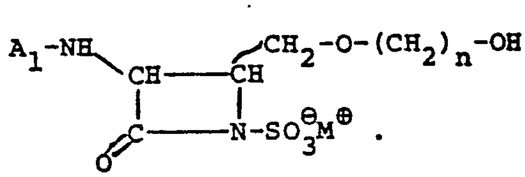

XVII

Conversion of a compound of formula XVII to a product of formula I can be accomplished using the methodology described above for the conversion of a compound of formula IV to a product of formula I, yielding first a compound having the formula

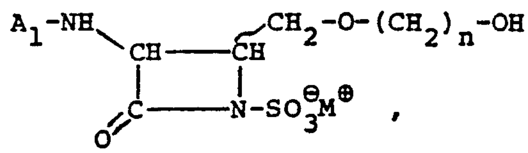

XVIII

then a zwitterion having the formula

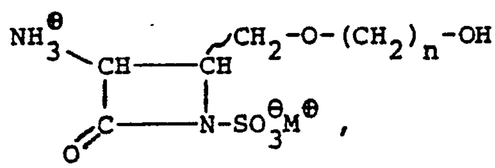

XIX

and finally the desired product of formula I.

To prepare those products of formula I wherein $R_2$ is $NH_2-(CH_2)_n-$ a compound of formula X wherein $R_2''$ is alkoxycarbonylalkyl is selectively reduced (using, for example, a chemical reducing agent such as sodium borohydride) to yield a compound having the formula

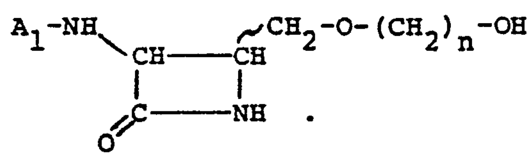

XX

The hydroxyl group of a compound of formula XX can be converted to a leaving group by reaction with a reagent such as *p*-toluenesulfonyl chloride and then reacted with sodium azide to yield a compound having the formula

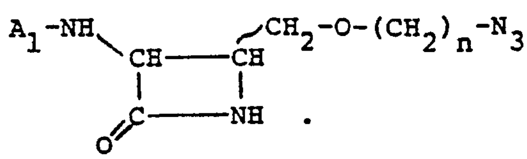

XXI

Hydrogenation of a compound of formula XXI followed by protection of the resulting amino group yields the corresponding compound having the formula

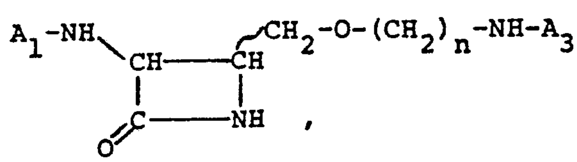

XXII

wherein $A_3$ is a nitrogen protecting group. It is important that $A_1$ and $A_3$ be different protecting groups.

Introduction of a sulfo substituent to the 1-position of a compound of formula XXII can be accomplished using the procedures described above for the introduction of a sulfo substituent onto a compound of formula IV. The resulting compound has the formula

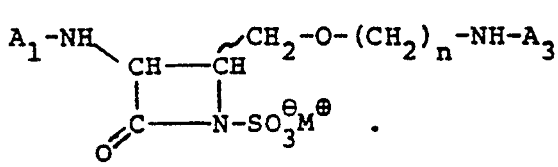

XXIII

Deprotection of the 3-amino group of a compound of formula XXIII followed by acylation of the resulting compound yields a compound having the formula

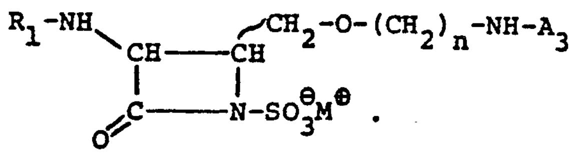

XXIV

Deprotection of the amino group in the 4-position yields the desired product of formula I.

The β-lactams of formula I wherein R is methoxy can be prepared from the corresponding compound having the formula

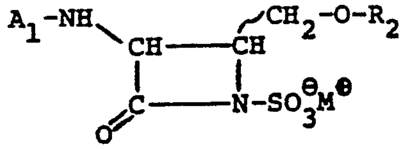

XXV

wherein $A_1$ is preferably benzyloxycarbonyl. Halogenation of the amide nitrogen of a compound of the above formula yields the corresponding compound having the formula

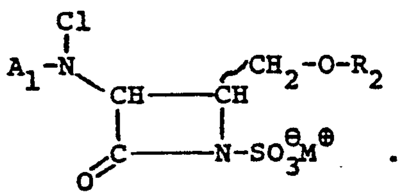

XXVI

Reaction of an intermediate of formula XXVI with a methoxylating agent, *e.g.,* an alkali metal methoxide yields a compound having the formula

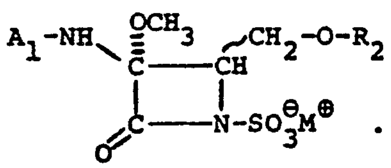

XXVII

The reaction can be run in an organic solvnt, *e.g.,* a polar organic solvent such as dimethylformamide, at a reduced temperature.

Removal of the amino protecting group from a compound of formula XXVII followed by acylation yields the desired product of formula I wherein R is methoxy.

An alternative synthesis for preparing the compounds of formula I wherein R is methoxy utilizes a compound having the formula

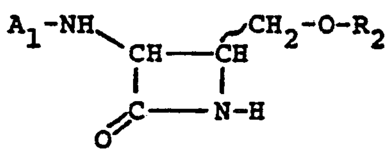

XXVIII

as a starting material ($A_1$ is preferably benzyloxycarbonyl). Halogenation of a compound of formula XXVIII yields the corresponding compound having the formula

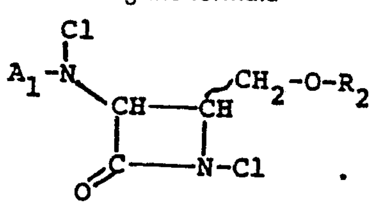

XXIX

Methoxylation of a compound of formula XXIX followed by reduction with a reagent such as trimethylphosphite yields an intermediate having the formula

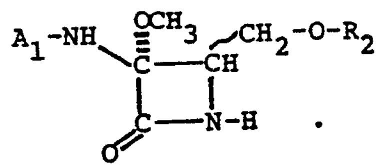

XXX

A sulfo group can be introduced in the 1-position of a compound of formula XXX using the above-described techniques. Removal of the nitrogen protecting group, followed by acylation, yields the desired product of formula I wherein R is methoxy.

In the above methoxylation procedures, when $R_2$ is $NH_2—(CH_2)_n—$, it will also be necessary to protect and deprotect the free amino group.

Compounds of formula II can be prepared using art-recognized procedures; see, for example, United States patent 4,166,816, issued September 4, 1979.

Compounds of formula VII can be prepared by reacting a primary amine having the formula

$$A_2—NH_2 \qquad XXXI$$

with an aldehyde having the formula

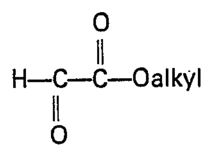

XXXII

to yield the corresponding Schiff base. A [2+2] cycloaddition reaction of the Schiff base with an activated form of α-phthalimidoacetic acid yields a compound having the formula

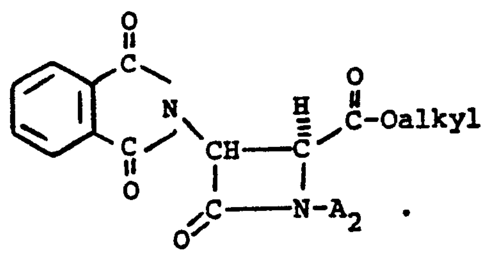

XXXIII

Treatment of a compound of formula XXXIII with base yields the corresponding 4α compound having the formula

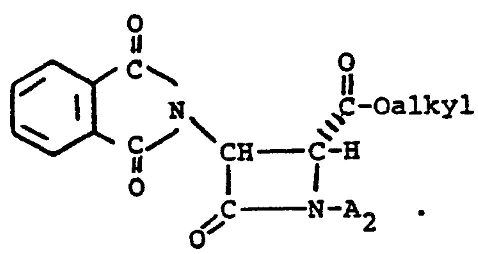

XXXIV

Reaction of a compound of formula XXXIII or XXXIV with a reagent such as methyl hydrazine (to cleave the phthaloyl group), followed by the introduction of an "$A_1$" protecting group on the 3-nitrogen substituent yields the corresponding compound of formula VII.

The following examples are specific embodiments of this invention.

Example 1

[3α(Z),4α]-3-[[(2-Amino-4-thiazolyl)(methoxyimino)-acetyl]amino]-4-(methoxymethyl)-2-oxo-1-azetidinesulfonic acid, potassium salt

A) (*cis*)-3-[[(Phenylmethoxy)carbonyl]amino]-4-methoxycarbonyl-2-oxo-1-(4-methoxyphenyl)azetidine

A solution of 12.5 g of (*cis*)-3-amino-4-methoxycarbonyl-2-oxo-1-(4-methoxyphenyl)azetidine prepared from the reaction of 7.7 ml of N-methylhydrazine with 18.65 g of (*cis*)-3-phthalimido-4-methoxycarbonyl-2-oxo-1-(4-methoxyphenyl)azetidine in 200 ml of dichloromethane is cooled to 0°C, and treated with diisopropylethylamine (17 ml) followed by benzyl chloroformate (7 ml) dropwise. The reaction is stirred at 0°C for 30 minutes, then at ambient temperature for 1.5 hours. The mixture is washed with two 300 ml portions of 0.5 M, 4.5 potassium dihydrogen phosphate buffer, two 300 ml portions of 5% sodium

bicarbonate and one 300 ml portion of saturated sodium chloride. The solution is then dried over sodium sulfate and concentrated *in vacuo* to a foam. Trituration with ether and filtration affords 9.9 g of the title compound as a solid.

B) (*cis*)-3-[[(Phenylmethoxy)carbonyl]amino]-4-(hydroxymethyl)-2-oxo-1-(4-methoxyphenyl)azetidine

A solution of 20.0 g of (*cis*-3-[[(phenylmethoxy)carbonyl]amino]-4-methoxycarbonyl-2-oxo-1-(4-methoxyphenyl)azetidine in 400 ml of tetrahydrofuran is cooled to 0°C under nitrogen, and a solution of 13.85 g of sodium borohydride in 160 ml of water is added over 20 minutes. The reaction mixture is stirred at 0°C for 20 minutes and then at ambient temperature for 1.25 hours. Over the next 60 minutes an additional 11.86 g of sodium borohydride is added as a solid in two equal portions, with preliminary cooling of the reaction mixture to 0°C, and subsequent warming to ambient temperature. The excess sodium borohydride is decomposed by adding 3N hydrochloric acid to pH 4.0 while cooling in an ice bath. This mixture is poured into 1500 ml of 50% brine and extracted with four 800 ml portions of methylene chloride. The combined organic extracts are washed with two 550 ml portions of 5% sodium bicarbonate and 500 ml of brine, then dried (sodium sulfate) and concentrated *in vacuo* to 17.71 g of a solid. The solid is boiled in 25 ml of ethyl acetate, cooled and filtered to yield 14.15 g of the title compound as a solid.

C) (*cis*)-3-[[(Phenylmethoxy)carbonyl]amino]-4-(methoxymethyl)-2-oxo-1-(4-methoxyphenyl)azetidine

In a 1000 ml 3-necked round bottom flask under nitrogen, 2.00 g of (*cis*)-3-[[(phenylmethoxy)carbonyl]-amino]-4-(hydroxymethyl)-2-oxo-1-(4-methoxyphenyl)azetidine is suspended in 160 ml of methylene chloride and cooled in an ice bath. To this suspension is added 0.07 ml of boron trifluoride etherate. An ethereal solution of diazomethane (prepared from 0.23 mole of N-methyl-N'-nitro-N-nitrosoguanidine in 400 ml of ether) is added in four equal portions, and the mixture is stirred under nitrogen for two hours with gradual warming to ambient temperature. The reaction mixture is then diluted with 800 ml of methylene chloride and washing with two 500 ml portions of saturated sodium bicarbonate and 500 ml of water. A waxy solid (presumably polymethylene) is removed by filtration, and the supernatant is washed with 500 ml of brine, dried (sodium sulfate), and concentrated *in vacuo* to a waxy solid. Trituration with ether and filtration affords 1.055 g of the title compound as solid.

D) (*cis*)-3-[[(Phenylmethoxy)carbonyl]amino]-4-(methoxymethyl)-2-azetidinone

A solution of 1.021 g of (*cis*)-3-[[(phenylmethoxy)carbonyl]amino]-4-(methoxymethyl)-2-oxo-1-(4-methoxyphenyl)azetidine in 35 ml of acetonitrile is cooled to −5°C. To this is added quickly a solution of 4.54 g of ceric ammonium nitrate in 40 ml of water. The resulting red solution is stirred at −5°C—0°C for 35 minutes, diluted with 200 ml of water, and extracted with three 75 ml portions of ethyl acetate. The combined organic extracts are washed with 100 ml of 5% sodium bicarbonate, then with 100 ml portions of 50% saturated sodium sulfite until the aqueous phase remains colorless. The organic solution is then washed with 100 ml of 5% sodium bicarbonate and 100 ml of brine and swirled over Darco G—60 for 30 minutes. This suspension is filtered and concentrated to a solid. This crude material is triturated with ether and filtered to yield 0.420 g of an off-white solid. Chromatography of this material on a "Prep Pak" silica gel column (Waters Prep. 500 HPLC) using 80% ethyl acetate/hexane affords 0.240 g of the title compound as a solid.

E) (*cis*)-3-[[(Phenylmethoxy)carbonyl]amino]-4-(methoxymethyl)-2-oxo-1-azetidinesulfonic acid, tetrabutylammonium salt

To a solution of 0.050 g of (*cis*)-3-[[(phenylmethoxy)carbonyl]amino]-4-(methoxymethyl)-2-azetidinone in 0.5 ml of dimethylformamide is added 0.120 g of pyridine-sulfur trioxide. This solution is stirred at ambient temperature under nitrogen for 16 hours. The reaction mixture is then poured into 60 ml of 0.5 M, pH 5.5 potassium dihydrogen phosphate buffer, and washed with four 20 ml portions of methylene chloride. To the aqueous layer is added 0.064 g of tetra-n-butylammonium hydrogen sulfate. The cloudy aqueous layer is then extracted with five 20 ml portions of methylene chloride, and the combined organic extracts are washed with 30 ml of brine, dried (sodium sulfate) and concentrated *in vacuo* to 0.087 g of the title compound as a 77% by weight solution in dimethylformamide (by n.m.r.).

F) [3α(Z),4α]-3-[[(2-amino-4-thiazolyl)(methoxyimino)acetyl]amino]-4-(methoxymethyl)-2-oxo-1-azetidine-sulfonic acid, potassium salt

A solution of 0.087 g of (*cis*)-3-[[(phenylmethoxy)carbonyl]amino]-4-(methoxymethyl)-2-oxo-1-azetidinesulfonic acid, tetrabutylammonium salt in 1 ml of dimethylformamide is hydrogenated over 0.044 g of 10% palladium on carbon for 3.25 hours at 1 atmosphere. A solution of 0.033 g of (Z)-2-amino-α-(methoxyimino)-4-thiazoleacetic acid and 0.025 g of 1-hydroxybenzotriazole hydrate in 0.6 ml of dimethylformamide is treated with 0.034 g of N,N'-dicyclohexylcarbodiimide. This suspension is stirred under nitrogen at ambient temperature for 25 minutes. The hydrogenation mixture (as described above) is added by pipette and rinsed in with 0.05 ml of dimethylformamide. This mixture is stirred for 18 hours at ambient temperature under nitrogen. The catalyst is removed by filtration (a small amount remains suspended in the solution) and washed with dimethylformamide. The supernatant is concentrated *in vacuo* at 30°C. Acetone is added to the residue to precipitate dicyclohexylurea. The acetone is removed from the solid, and

the solid is washed twice with acetone. The combined acetone solutions are treated with 0.051 g of potassium perfluorobutanesulfonate to precipitate a solid. The mother liquor is removed and ether is added to precipitate a second crop of solid. The solid is washed with ether and hexane and dried *in vacuo* at ambient temperature. The two crops are combined to yield 0.061 g of crude product. This material is dissolved in water, applied to a column of 60 ml of HP20AG, and eluted with water. Concentration of the appropriate fractions yields a glass-like material, which upon trituration with acetonitrile and evaporation, affords 0.027 g of the title compound as a solid.

Anal. for $C_{11}H_{14}N_5O_7S_2K \cdot 0.5\ H_2O$:

| | | | |
|---|---|---|---|
| Calc'd: | C, 29.99; | H, 3.44; | N, 15.90 |
| Found: | C, 29.90; | H, 3.46; | N, 15.30 |

Example 2

[[[[(4-Ethyl-2,3-dioxo-4-piperazinyl)carbonyl]amino]phenylacetyl]amino]-4-(methoxymethyl)-2-oxo-1-azetidinesulfonic acid, potassium salt

A solution of 0.150 g of (*cis*)-3-[[(phenylmethoxy)carbonyl]amino]-4-(methoxymethyl)-2-oxo-1-azetidinesulfonic acid, tetrabutylammonium salt (see example 1E) in 1.5 ml of methanol is hydrogenated over 0.075 g of 10% palladium on carbon for 1 hour at 1 atmosphere. The catalyst is removed by filtration and washed with methanol. The combined methanol solutions are concentrated *in vacuo*, and the resulting residue is dissolved in 10.5 ml of acetonitrile and cooled to −20°C. To this cold solution is added 0.095 ml of dry pyridine, followed by a solution of 0.132 g of (R)-α-[[(4-ethyl-2,3-dioxo-1-piperazinyl)-carbonyl]amino]phenylacetyl chloride in 1.5 ml of acetonitrile. This solution is stirred at −20°C under nitrogen for 1.5 hours, and then diluted with 10 ml of 0.5 M, pH 5.5 potassium dihydrogen phosphate buffer and concentrated *in vacuo* to remove the acetonitrile. The aqueous residue is extracted with three 12 ml portions of methylene chloride, and the combined extracts are dried (sodium sulfate) and concentrated *in vacuo* to 0.155 g of the title compound as the tetrabutylammonium salt.

A solution of 0.155 g of the tetrabutylammonium salt in 1.5 ml of acetone is treated with 0.070 g of potassium perfluorobutanesulfonate to precipitate a solid. Addition of ether to this suspension produces more precipitate. The solid is separated from the supernatant and washed with ether (five times) and hexane and dried *in vacuo* to afford 0.093 g of the title compound as an impure solid. This material is chromatographed on 50 ml of HP20AG using water, 5% acetone in water, and 10% acetone in water. The combination and concentration of the appropriate fractions yields a waxy solid which upon trituration with hexane and evaporation, affords 0.046 g of the title compound as a solid.

Anal. for $C_{20}H_{24}N_5O_9SK \cdot H_2O$:

| | | | | |
|---|---|---|---|---|
| Calc'd: | C, 42.31; | H, 4.63; | N, 12.34; | S, 5.65 |
| Found: | C, 42.28; | H, 4.74; | N, 12.33; | S, 5.46 |

Example 3

[3α(Z),4α]-2-[[[1-(2-Amino-4-thiazolyl)-2-[[4-(methoxymethyl)-2-oxo-1-sulfo-3-azetidinyl]amino]-2-oxoethylidene]amino]oxy]-2-methylpropanioc acid, dipotassium salt

A solution of 0.216 g of (*cis*)-3-[[(phenylmethoxy)carbonyl]amino]-4-(methoxymethyl)-2-oxo-1-azetidinesulfonic acid, tetrabutylammonium salt (see example IE) in 2 ml of dimethylformamide is hydrogenated over 0.109 g of 10% palladium on carbon for 4 hours at 1 atmosphere. To a solution of 0.188 g of (Z)-2-amino-α-[(1-diphenylmethoxycarbonyl-1-methylethoxy)imino]-4-thiazoleacetic acid and 0.062 g of hydroxybenzotriazole monohydrate in 1.3 ml of dimethylformamide is added 0.084 g of dicyclohexylcarbodiimide. This resulting suspension is stirred at ambient temperature under nitrogen for 30 minutes, and the hydrogenation mixture (as described above) is added by pipette. This mixture is stirred at ambient temperature under nitrogen for 18 hours. The catalyst is removed by filtration (a small amount remains suspended in solution) and washed with dimethylformamide. The supernatant is concentrated *in vacuo* at 30°C, and the residue is triturated with acetone to precipitate dicyclohexylurea. This solid is removed from the acetone solution and washed twice with acetone. The combined acetone solutions are treated with 0.125 g of potassium perfluorobutanesulfonate to precipitate a solid. Ether is added to precipitate more solid, which is removed, washed with ether and hexane, and dried *in vacuo* to yield 0.172 g of crude coupling product.

A suspension of 0.051 g of this crude solid in 0.35 ml of anisole is cooled to −20°C, and 0.77 ml of cold (−10°C) distilled trifluoroacetic acid is added dropwise. The solution is then stirred at −15°C for 1.5 hours. The addition of 3.5 ml of ether and 1.8 ml of hexane to the solution results in immediate precipitation. This resulting suspension is stirred at −10°C for 15 minutes, then at ambient temperature for 15 minutes. The suspension is centrifuged and the supernatant removed. The remaining waxy solid is washed three times with ether, dried *in vacuo* at ambient temperature, and suspended in 5 drops of water. The pH of this suspension is adjusted to 5.5 with 0.5 N potassium hydroxide and 1 N hydrochloric acid. The resulting clear solution is applied to 26 ml of HP20AG and eluted with water. Combination and concentration of the appropriate fractions yields a pale glass which upon trituration and evaporation with acetonitrile and evaporation with hexane, yields 0.011 g of the title compound as a solid. This is combined with 0.017 g

more of the title compound which is prepared in an identical manner, with the exception that the pH is adjusted to 5.5 using only 0.5 N potassium hydroxide. The total yield of the title compound is 0.028 g.

Anal. for $C_{14}H_{11}N_5O_9S_2K_2$ 1.85 $H_2O$:
Calc'd: C, 29.24; H, 3.62; N, 12.18
Found: C, 29.24; H, 3.47; N, 12.36

Examples 4—5

Following the procedure of example 1, but substituting the compound listed in column I for diazomethane, yields the compound listed in column II.

| | Column I | Column II |
|---|---|---|
| 4. | diazoethane | [3α(Z),4α]-3-[[(2-amino-4-thiazolyl)(methoxyimino)-acetyl]amino]-4-(ethoxymethyl)-2-oxo-1-azetidine-sulfonic acid, potassium salt |
| 5. | diazobutane | [3α(Z),4α]-3-[[(2-amino-4-thiazolyl)(methoxyimino)acetyl]-amino]-4-(butoxymethyl)-2-oxo-1-azetidinesulfonic acid, potassium salt |

Example 6

[3α(Z),4α]-[[[3-[[2-Amino-4-thiazolyl)(methoxyimino)acetyl]amino]-2-oxo-1-sulfo-4-azetidinyl]methyl]oxy]acetic acid, potassium salt, methyl ester

A) (*cis*)-[[[3-[[(Phenylmethoxy)carbonyl]amino]-2-oxo-1-(4-methoxyphenyl)-4-azetidinyl]methyl]oxy]acetic acid, methyl ester

A solution of (*cis*) - 3 - [[(phenylmethoxy)carbonyl]amino - 4 - (hydroxymethyl) - 2 - oxo - 1 - (4 - methoxyphenyl)azetidine (500 mg; see example 1B) and rhodium (II) acetate (2 ml) in benzene is treated dropwise with a solution of ethyl diazoacetate (160 mg) in benzene. After stirring overnight, the mixture is concentrated and chromatographed to yield the title compound.

B) [3α(Z),4α]-[[[3-[[2-Amino-4-thiazolyl)(methoxyimino)acetyl]amino]-2-oxo-1-sulfo-4-azetidinyl]methyl]oxy]acetic acid, potassium salt, methyl ester

Following the procedure of example 1, parts D—F, but substituting (*cis*)-[[[3-[[phenylmethoxy)-carbonyl]amino]-2-oxo-1-(4-methoxyphenyl)-4-azetidinyl]methyl]oxy]acetic acid, methyl ester for (*cis*)-3-[[(phenylmethoxy)carbonyl]amino]-4-(methoxymethyl)-2-oxo-1-(4-methoxyphenyl)azetidine, yields the title compound.

Example 7

[3α(Z),4α]-3-[[(2-Amino-4-thiazolyl)(methoxyimino)acetyl]amino]-4-(phenoxymethyl)-2-oxo-1-azetidinesulfonic acid, potassium salt

A (*cis*)-3-(*t*-Butoxy)carbonyl]amino]-4-methoxycarbonyl-2-oxo-1-(4-methoxyphenyl)azetidine

A solution of 9.75 g of (*cis*)-3-amino-4-methoxycarbonyl-2-oxo-1-(4-methoxyphenyl)azetidine prepared from the reaction of 6.2 ml of N-methylhydrazine with 15.00 g of (*cis*)-3-phthalimido-4-methoxycarbonyl-2-oxo-1-(4-methoxyphenyl)azetidine in 200 ml of dichloromethane is cooled to 0°C, and 9.61 g of di-*t*-butyl pyrocarbonate is added. The solution is allowed to warm to ambient temperature and is stirred for 24 hours. At this time an additional 8.74 g of di-*t*-butylpyrocarbonate is added. The solution is stirred at ambient temperature for 2 hours, and washed with three 150 ml portions of 0.5 M, pH 4.5 potassium dihydrogen phosphate buffer, one 150 ml portion of 5% sodium bicarbonate and one 150 ml portion of saturated sodium chloride. The solution is then dried over sodium sulfate and concentrated *in vacuo* to a waxy solid. Trituration with ether and filtration affords 9.67 g of the title compound as a solid.

B) (*cis*)-3-[[(*t*-Butoxy)carbonyl]amino]-4-methoxycarbonyl-2-azetidinone

A solution of 5.00 g of (*cis*)-3-[[(*t*-butoxy)carbonyl]amino]-4-methoxycarbonyl-2-oxo-1-(4-methoxyphenyl)azetidine in 190 ml of acetonitrile is cooled to −7°C, and to this is added quickly a solution of 23.49 g of ceric ammonium nitrate in 200 ml of water. This solution is stirred at 0°—5° for 40 minutes, diluted with 850 ml of water, and extracted with three 400 ml portions of ethyl acetate. The combined ethyl acetate extracts are washed with 400 ml of 5% sodium bicarbonate and the aqueous layer is back-extracted with 100 ml of ethyl acetate. The combined ethyl acetate layers are then washed with 400 ml portions of 50% saturated sodium sulfite until the aqueous phase remains colorless. The organic solution is washed with 400 ml of 5% sodium bicarbonate, 400 ml of 50% saturated sodium sulfite, 300 ml of saturated sodium chloride, and swirled over Darco G—60 for 1.5 hours. The Darco G—60 is removed by filtration through a pad of Celite. The organic solution is concentrated *in vacuo* to a solid. Trituration with ether and filtration yields 2.00 g of the title compound as a white solid.

C) (*cis*)-3-[[(*t*-Butoxy)carbonyl]amino]-4-(hydroxymethyl)-2-azetidinone

A solution of 1.98 g of (*cis*)-3-[[(*t*-butoxy)carbonyl]amino]-4-methoxycarbonyl-2-azetidinone in 20 ml of tetrahydrofuran is cooled to 0°C, and a solution of 0.739 g of sodium borohydride in 12 ml of water is added dropwise. The resulting mixture is stirred at 0°C for 20 minutes, and then at ambient temperature for 1.75 hours. The excess borohydride is decomposed by adding acetic acid to pH 4.3 while cooling in an ice bath. The acidic solution is diluted with 150 ml of saturated sodium chloride and extracted with three 75 ml portions of ethyl acetate. The combined organic layers are washed with two 200 ml portions of 5% sodium bicarbonate and 100 ml of saturated sodium chloride, dried over sodium sulfate, and concentrated *in vacuo* to 0.478 g of solid. The sodium bicarbonate wash is then back-extracted with four 75 ml portions of ethyl acetate. These combined organic layers are dried over sodium sulfate and concentrated *in vacuo* to 0.283 g of solid. The two crops of solid are combined to afford 0.761 g of the title compound.

D) (*cis*)-3-[[(*t*-Butoxy)carbonyl]amino]-4-[(4-methylphenyl)sulfonoxymethyl]-2-azetidinone

A solution of 0.700 g of (*cis*)-3-[[(*t*-butoxy)carbonyl]amino]-4-(hydroxymethyl)-2-azetidinone in 7 ml of pyridine is cooled to 0°C under nitrogen, and 1.11 g of *p*-toluenesulfonyl chloride is added as a solid. The solution is stirred under nitrogen at 5°C for 18 hours, and then poured into 300 ml of cold (0°C) ethyl acetate. This solution is washed with two 75 ml portions of cold (0°C) 0.5 N hydrochloric acid, 150 ml of cold (0°C) 50% saturated sodium chloride, and dried over sodium sulfate. The dry organic solution is then concentrated *in vacuo* to a solid. Trituration with ether and filtration, followed by additional ether washes affords 1.046 g of the title compound.

E) (*cis*)-3-[[(*t*-Butoxy)carbonyl]amino]-4-(phenoxymethyl)-2-oxo-1-azetidine

In a dry flask under argon, 0.446 g of sodium hydride (as a 50% dispersion in mineral oil) is washed with three 1.5 ml portions of hexane to remove the mineral oil. The sodium hydride is suspended in 5 ml of dimethylformamide and cooled to 0°C. To this is added dropwise 3.1 ml of a 3M solution of phenol in dimethylformamide, and the mixture is stirred at 0°C for 10 minutes. A solution of 3.75 g of (*cis*)-3-[[(*t*-butoxy)carbonyl]amino]-4-[(4-methylphenyl)sulfonoxymethyl]-2-azetidinone in 10 ml of dimethylformamide is added dropwise. The reaction mixture is stirred at 0°C for 20 minutes and poured into a cold (0°C) mixture of 400 ml of ethyl acetate and 500 ml of water. The layers are separated, and the organic phase is washed with 400 ml of cold (0°C) 50% saturated sodium bicarbonate and 400 ml of saturated sodium chloride, and then dried over sodium sulfate and concentrated *in vacuo* to yield the title compound.

F) (*cis*)-3-[[(*t*-Butoxy)carbonyl]amino]-4-(phenoxymethyl)-2-oxo-1-azetidinesulfonic acid, tetrabutylammonium salt

Following the procedure of Example 1E, but substituting (*cis*)-3-[[(*t*-butoxy)carbonyl]amino]-4-(phenoxymethyl)-2-oxo-1-azetidine for (*cis*)-3-[[(phenylmethoxy)carbonyl]amino]-4-(methoxymethyl)-2-azetidinone, yields the title compound.

G) (*cis*-3-Amino]-4-(phenoxymethyl)-2-oxo-1-azetidinesulfonic acid

A solution of 1.0 g of (*cis*)-3-[[(*t*-butoxy)carbonyl)amino-4-(phenoxymethyl)-2-oxo-1-azetidinesulfonic acid, tetrabutylammonium salt in 10 ml of 98% formic acid is stirred at ambient temperature for 1 hour and diluted with 10 ml of dichloromethane to precipitate a solid. The solid is removed from the supernatant, washed with three 10 ml portions of dichloromethane, and dried *in vacuo* at ambient temperature for 18 hours.

H) [3α(Z),4α]-3-[[(2-Amino-4-thiazolyl)(methoxyimino)acetyl]amino]-4-(phenoxymethyl)-2-oxo-1-azetidinesulfonic acid, potassium salt

To a solution of 0.123 g of (Z)-2-amino-α-(methoxyimino)-4-thiazoleacetic acid and 0.093 g of 1-hydroxybenzotriazole hydrate in 2.5 ml of dimethylformamide is added 0.126 g of N,N'-dicyclohexylcarbodiimide. This mixture is stirred at ambient temperature under argon for 30 minutes, and 0.1 g of (*cis*)-3-amino-4-(phenoxymethyl)-2-oxo-1-azetidinesulfonic acid and 0.09 ml of triethylamine are added. The resulting mixture is stirred at ambient temperature for about 12 hours, and concentrated *in vacuo* at 32°C. Acetone is added to the residue to precipitate dicyclohexylurea. The acetone is removed from the solid which is washed with additional acetone. The combined acetone solutions are treated with .187 g of potassium perfluorobutanesulfonate, followed by ether. The resulting precipitate is washed with ether and hexane and dried *in vacuo*. The crude material is chromatographed on HP—20 AG and eluted with water. Concentration of the appropriate fractions yields the title compound.

**Claims**

1. A β-lactam having the formula

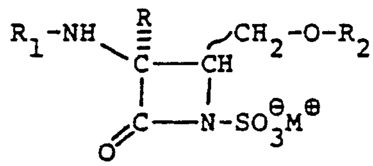

wherein

R is hydrogen or methoxy;

$R_1$ is acyl as commonly employed in β-lactam antibiotics;

$R_2$ is alkyl, arylalkyl, carboxyalkyl, alkoxycarbonylalkyl, $NH_2—(CH_2)_n—$, $HO—(CH_2)_n—$ or aryl, wherein alkyl and alkoxy are straight or branched chain groups having 1 to 10 carbon atoms; n is 2, 3 or 4; and aryl is phenyl or phenyl substituted with 1, 2 or 3 amino ($—NH_2$), halogen, hydroxyl, trifluoromethyl, alkyl (of 1 to 4 carbon atoms), alkoxy (of 1 to 4 carbon atoms) or carboxyl groups; and

M is hydrogen or $M^{\oplus}$ is a cation;

with the proviso that if M is hydrogen the $R_1$ or $R_2$ group contains a basic function.

2. A β-lactam in accordance with claim 1 wherein R is hydrogen.

3. A β-lactam in accordance with claim 1 wherein R is methoxy.

4. A β-lactam in accordance with claim 1 wherein $R_2$ is alkyl, and $R_1$ has a basic function when M is hydrogen.

5. A β-lactam in accordance with claim 1 wherein $R_2$ is arylalkyl, and $R_1$ has a basic function when M is hydrogen.

6. A β-lactam in accordance with claim 1 wherein $R_2$ is carboxyalkyl, and $R_1$ has a basic function when M is hydrogen.

7. A β-lactam in accordance with claim 1 wherein $R_2$ is alkoxycarbonylalkyl, and $R_1$ has a basic function when M is hydrogen.

8. A β-lactam in accordance with claim 1 wherein $R_2$ is $NH_2—(CH_2)_n—$.

9. A β-lactam in accordance with claim 1 wherein $R_2$ is $HO—(CH_2)_n—$.

10. A β-lactam in accordance with claim 1 wherein $R_2$ is aryl, and $R_1$ has a basic function when M is hydrogen.

11. The β-lactam in accordance with claim 1 [3α(Z),4α]-3-[[(2-amino-4-thiazolyl)(methoxyimino)-acetyl]amino)-4-(methoxymethyl)-2-oxo-1-azetidinesulfonic acid, potassium salt.

12. The β-lactam in accordance with claim 1 [[[[(4-ethyl-2,3-dioxo-4-piperazinyl)carbonyl]-amino]phenylacetyl]amino]-4-(methoxymethyl)-2-oxo-1-azetidinesulfonic acid, potassium salt.

13. The β-lactam in accordance with claim 1 [3α(Z),4α]-2-[[[1-(2-amino-4-thiazolyl)-2-[[4-(methoxymethyl)-2-oxo-1-sulfo-3-azetidinyl]amino]-2-oxoethylidene]amino]oxy]-2-methylpropanoic acid, dipotassium salt.

**Patentansprüche**

1. ß-Lactam mit der Formel

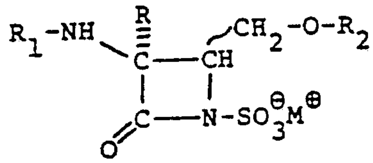

in der

R ein Wasserstoffatom oder eine Methoxygruppe ist,

$R_1$ eine in der Chemie der ß-Lactam-Antibiotika üblicherweise verwendete Acylgruppe bedeutet,

$R_2$ einen Alkyl-, Arylalkyl-, Carboxyalkyl- oder Alkoxycarbonylalkyl-Rest, einen Rest $NH_2—(CH_2)_n—$, oder $HO—(CH_2)_n$ oder einen Arylrest bedeutet, wobei die Alkyl- oder Alkoxyreste geradkettig oder verzweigt sind und 1 bis 10 Kohlenstoffatome haben,

n den Wert 2, 3 oder 4 hat,

der Arylrest aus einer Phenylgruppe besteht oder aus einer Phenylgruppe, die mit 1 2 oder 3 Aminogruppen ($—NH_2$), Halogenatomen, Hydroxyl- oder Trifluormethylgruppen, Alkylresten mit 1 bis 4 Kohlenstoffatomen oder Alkoxyresten mit 1 bis 4 Kohlenstoffatomen oder Carboxylgruppen substituiert ist; und der

M ein Wasserstoffatom oder $M^{\oplus}$ ein Kation ist, mit der Maßgabe, daß, wenn M ein Wasserstoffatom ist, dann

$R_1$ oder $R_2$ eine basische Funktion trägt.

2. ß-Lactam nach Anspruch 1, in dem R ein Wasserstoffatom ist.

3. ß-Lactam nach Anspruch 1, in dem R eine Methoxygruppe ist.

4. ß-Lactam nach Anspruch 1, in dem $R_2$ ein Alkylrest ist, und $R_1$ eine basische Funktion trägt, wenn M ein Wasserstoffatom ist.

5. ß-Lactam nach Anspruch 1, in dem $R_2$ ein Arylalkylrest ist, und $R_1$ eine basische Funktion trägt, wenn M ein Wasserstoffatom ist.

6. ß-Lactam nach Anspruch 1, in dem $R_2$ ein Carboxyalkylrest ist, und $R_1$ ein basische Funktion trägt, wenn M ein Wasserstoffatom ist.

7. ß-Lactam nach Anspruch 1, in dem $R_2$ ein Alkoxycarbonylalkylrest ist, und $R_1$ eine basische Funktion trägt, wenn M ein Wasserstoffatom ist.

8. ß-Lactam nach Anspruch 1, in dem $R_2$ ein Rest $NH_2—(CH_2)_n—$ ist.

9. ß-Lactam nach Anspruch 1, in dem $R_2$ ein Rest $HO—(CH_2)_n—$ ist.

10. ß-Lactam nach Anspruch 1, in dem $R_2$ ein Arylrest ist, und $R_1$ eine basische Funktion trägt, wenn M ein Wasserstoffatom ist.

11. β-Lactam nach Anspruch 1, nämlich das Kalium der [3α(Z),4α]-3-[[(2-Amino-4-thiazolyl)(methoxyimino)acetyl]amino]-4-(methoxymethyl)-2-oxo-1-azetidinsulfonsäure.

12. ß-Lactam nach Anspruch 1, nämlich das Kalium der [[[[(4-Äthyl-2,3-dioxo-4-piperazinyl)-carbonyl]-amino]phenylacetyl]-amino]-4-(methoxymethyl)-2-oxo-1-azetidinsulfonsäure.

13. ß-Lactam nach Anspruch 1, nämlich das Dikaliumsalz der [3α(Z),4α]-2-[[[(1-(2-Amino-4-thiazolyl)-2-[[4-(methoxymethyl)-2-oxo-1-sulfo-3-azetidinyl]-amino]-2-oxoäthyliden]-amino]-oxy]-2-methyl-propionsäure.

**Revendications**

1. β-lactame ayant pour formule

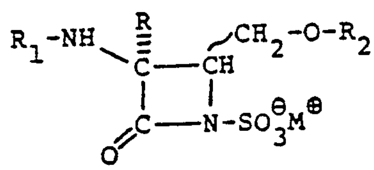

dans laquelle R est un atome d'hydrogène ou un groupement méthoxy;

$R_1$ est un radical acyle tel qu'il est communément employé dans les antibiotiques de la famille des β-lactames;

$R_2$ est un radical alkyle, arylalkyle, carboxyalkyle, alcoxycarbonylalkyle, $NH_2—(CH_2)_n—$, $HO—(CH_2)_n—$ ou aryle, les radicaux alkyle et alcoxy étant des groupements à chaîne droite ou ramifiée ayant de 1 à 10 atomes de carbone; n étant égal à 2, 3 ou 4; et le radical aryle étant un radical phényle éventuellement substitué par 1, 2 ou 3 groupements amine ($—NH_2$), atomes d'halogène, groupements hydroxyle, ou radicaux trifluorométhyle, alkyle (de 1 à 4 atomes de carbone), alcoxy (de 1 à 4 atomes de carbone) ou carboxyle; et

M est un atome d'hydrogène ou $M^{\oplus}$ est un cation; avec cette réserve que, si M est un atome d'hydrogène, le groupement $R_1$ ou $R_2$ contient une fonction basique.

2. β-lactame selon la revendication 1, dans la formule duquel R est un atome d'hydrogène.

3. β-lactame selon la revendication 1, dans la formule duquel R est un groupement méthoxy.

4. β-lactame selon la revendication 1, dans la formule duquel $R_2$ est un radical alkyle, et $R_1$ a une fonction basique quand M est un atome d'hydrogène.

5. β-lactame selon la revendication 1, dans la formule duquel $R_2$ est un radical arylalkyle, et $R_1$ a une fonction basique quand M est un atome d'hydrogène.

6. β-lactame selon la revendication 1, dans la formule duquel $R_2$ est un radical carboxyalkyle, et $R_1$ a une fonction basique quand M est un atome d'hydrogène.

7. β-lactame selon la revendication 1, dans la formule duquel $R_2$ est un radical alcoxycarbonylalkyle, et $R_1$ a une fonction basique quand M est un atome d'hydrogène.

8. β-lactame selon la revendication 1, dans la formule duquel $R_2$ est un groupement de formule $NH_2—(CH_2)_n—$.

9. β-lactame selon la revendication 1, dans la formule duquel $R_2$ est un groupement de formule $HO—(CH_2)_n—$.

10. β-lactame selon la revendication 1, dans la formule duquel $R_2$ est un radical aryle, et $R_1$ a une fonction basique quand M est un atome h'hydrogène.

11. β-lactame selon la revendication 1, qui est le sel de potassium de l'acide [3α(Z),4α]-3-[[(2-amino-4-thiazolyl)(méthoxyimino)acétyl]amino]-4-(méthoxyméthyl)-2-oxo-1-azétidinesulfonique.

12. β-lactame selon la revendication 1, qui est le sel de potassium de l'acide [[[[(4-éthyl-2,3-dioxo-4-pipérazinyl)carbonyl]amino]phénylacétyl]amino]-4-(méthoxyméthyl)-2-oxo-1-azétidinesulfonique.

13. β-lactame selon la revendication 1, qui est le sel dipotassique de l'acide [3α(Z),4α]-2-[[[(1-(2-amino-4-thiazolyl)-2-[[4-(méthoxyméthyl)-2-oxo-1-sulfo-3-azétidinyl]amino]-2-oxo-éthylidène]amino]oxy]-2-méthylpropanoïque.